# EUROPEAN PATENT SPECIFICATION

(11) **EP 3 646 832 B1**
(45) Date of publication and mention of the grant of the patent: **29.09.2021**
(21) Application number: 17928596.0
(22) Date of filing: 12.10.2017
(51) Int. Cl.: A61F 13/49, A61F 13/496, A61F 13/514

(54) **ABSORBENT ARTICLE**
ABSORBIERENDER ARTIKEL
ARTICLE ABSORBANT

(43) Date of publication of application: 06.05.2020
(73) Proprietor: Unicharm Corporation, Shikokuchuo-shi, Ehime 799-0111 (JP)
(72) Inventor: FUJII, Keishi, Kanonji-shi Kagawa 769-1602 (JP); UEDA, Masumi, Kanonji-shi Kagawa 769-1602 (JP)
(74) Representative: Dolleymores
(86) International application number: PCT/JP2017/037003
(87) International publication number: WO 2019/073572

(56) References cited:
- EP-A1- 2 186 495
- EP-A1- 2 258 327
- WO-A1-2017/175287
- WO-A2-96/31179
- JP-A- 2009 061 127
- JP-A- 2011 078 477
- JP-A- 2014 221 122

## Description

### Technical Field

The present invention relates to an absorbent article.

### Background

A disposable diaper and the like are known as an absorbent article that absorbs excrements such as urine. For example, PTL 1 discloses forming a plurality of through holes 25 penetrating in the thickness direction to improve air permeability of an exterior body 2 of a pull-on absorbent article 1A and to prevent stuffiness inside.

### Citation List

### Patent Literature

[PTL 1] Japanese Patent Application Publication No. 2015-107223 EP 2258327 A1 relates to an absorbent article having slits formed in a front waist region elastic member and/or in a rear waist region elastic member. The slits are stretched in a transverse direction under the effect of the waist region elastic member to form through-holes extending from a skin-facing side of the absorbent article to a garment facing side of the absorbent article.

### Summary

### Technical Problem

With the pull-on absorbent article 1A indicated in PTL 1 in the natural state, however, due to the through holes 25 being hidden with creases formed from the contraction of an elastic member 23, or the through holes 25 being contracted in a horizontal direction Y, there is a possibility that a wearer or a person putting on the pull-on absorbent article 1A cannot visually identify the through holes 25, and there is a possibility that good air permeability of the pull-on absorbent article 1A cannot be recognized from the outside.

This invention has been made in view of the above issues, and an object is to make it easy to give an impression of good air permeability of an absorbent article having through holes to a wearer of the absorbent article or a person putting on the absorbent article.

### Solution to Problem

The present invention provides the absorbent article of independent claim 1. The dependent claims specify preferred but optional features.

Other features of this invention will become clear from descriptions in this specification and attached drawings.

### Advantageous Effects of Invention

According to this invention, when wearing/putting on the absorbent article, when an elastic member is extended, holes can easily be visually identified. In particular, when the absorbent article is in a natural state, such as when the absorbent article is taken out of a package, the holes that were difficult to visually identify, due to creases formed by contraction of the elastic member, can be easily visually identified when the elastic member is extended when putting on the absorbent article, thus, when wearing/putting on the absorbent article a wearer or a person putting on the absorbent article is surprised by the presence of the holes and is more easily given an impression of good air permeability.

### Brief Description of Drawings

[Fig. 1] Fig. 1A is a schematic front view of a diaper 1 in a natural state seen from a front side as an example of an absorbent article of the present embodiment. Fig. 1B is a schematic rear view of a diaper 1 in a natural state seen from a rear side.
[Fig. 2] Fig. 2 is a plan view of a diaper 1 in an open stretched state seen from a skin side.
[Fig. 3] Fig. 3 is a sectional view along line III-III in Fig. 2.
[Fig. 4] Fig. 4 is a sectional view along line IV-IV in Fig. 2.
[Fig. 5] Fig. 5A is a schematic view of a section along Va-Va of a front-side waist part 20 in Fig. 1A. Fig. 5B is a schematic view of a section along Va-Va extended in the right-left direction from the state in Fig. 5A.
[Fig. 6] Fig. 6A is a schematic view of a section along Vb-Vb of a rear-side waist part 30 in Fig. 1B. Fig. 6B is a schematic view of a section along Vb-Vb extended in the right-left direction from the state in Fig. 6A.
[Fig. 7] Fig. 7 is a view showing an example of a method of a parent and the like putting on a diaper 1.
[Fig. 8] Fig. 8 is a schematic side view of a diaper 1 seen from a one end side in the right-left direction.
[Fig. 9] Fig. 9 is a view explaining an enlarged opening 50.

### Description of Embodiments

At least below matters will become clear from descriptions in this specification and attached drawings.

An absorbent article including an up-down direction, a right-left direction, and a front-rear direction, which intersect with each other, including: a front-side waist part; and a rear-side waist part, at least one of the front-side waist part and the rear-side waist part including an elastic member that expands and contracts in the right-left direction and holes penetrating in the front-rear direction, of the front-side waist part and the rear-side waist part, a waist part on another side with a greater contractile force in the right-left direction includes more of the holes than a waist part on one side with a smaller contractile force in the right-left direction.

According to such an absorbent article, when wearing/putting on the absorbent article, when the elastic member is extended, the holes can easily be visually identified. In particular, when the absorbent article is in a natural state, such as when the absorbent article is taken out of a package, the holes that were difficult to visually identify, due to creases formed by contraction of the elastic member, can be easily visually identified when the elastic member is extended when putting on the absorbent article, thus when wearing/putting on the absorbent article a wearer or a person putting on the absorbent article is surprised by the presence of the holes and is more easily given an impression of good air permeability.

With an absorbent article, wherein preferably both end parts of the front-side waist part in the right-left direction and both end parts of the rear-side waist part in the right-left direction are joined to each other.

According to such an absorbent article, a person putting on the absorbent article can more certainly visually identify the holes when putting on the absorbent article.

With the absorbent article, each of the front-side waist part and the rear-side waist part includes the holes.

According to such an absorbent article, air permeability in both the front and the back can be improved.

With an absorbent article, wherein preferably the waist part on the other side is the front-side waist part.

According to such an absorbent article, in the case that a person putting on the absorbent article to a wearer is putting on the absorbent article in the state facing the front of the wearer, the holes can be more easily visually identified.

With an absorbent article, wherein preferably the waist part on the other side is the rear-side waist part.

According to such an absorbent article, in the case that a person putting on the absorbent article to a wearer is putting on the absorbent article in the state facing the back of the wearer, the holes can be more easily visually identified.

With an absorbent article, wherein preferably the waist part on the other side includes a nonwoven fabric, in the up-down direction, a high-density region is included between each hole and the adjacent elastic member, and an outer region is included between the high-density region and the elastic member, the high-density region includes a predetermined length in the right-left direction, a density of fibers of the nonwoven fabric in the high-density region is higher than a density of fibers of the nonwoven fabric in the outer region.

According to such an absorbent article, because the rigidity of the high-density region is higher than the rigidity of the outer region, the shape of the holes can be easily maintained, and the holes can be easily visually identified.

With an absorbent article, wherein preferably the waist part on the other side includes a recessed and protruding shape in the front-rear direction, and the holes are provided to cross over a tip of a protruding part to protrude to a non-skin side.

According to such an absorbent article, the edge of the holes with high density of fibers decreases the possibility of contacting a skin of a wearer, and the touch of the absorbent article can be improved.

With an absorbent article, wherein preferably an absorbent body is included, and a length of a region in which the waist part on the other side overlaps with the absorbent body, in the up-down direction, is shorter than a length of a region in which the waist part on one side overlaps with the absorbent body.

According to such an absorbent article, with the waist part on the other side, the region overlapping the absorbent body with a relatively high rigidity in the up-down direction is short, thus elastic member can be easily extended in the right-left direction, and the holes that could not be easily visually identified in the natural state can be more easily visually identified when the absorbent article is put on/worn.

With an absorbent article, wherein preferably an absorbent body is included, and a number of the holes in a region, of the waist part on the other side, overlapping the absorbent body in the up-down direction is greater than a number of the holes in a region, of the waist part on the other side, not overlapping the absorbent body in the up-down direction.

According to such an absorbent article, the region overlapping the absorbent body in the up-down direction is a region that has little possibility of being held by hands of a person putting on the absorbent article, in a case where the person putting on the absorbent article puts on the absorbent article by holding an upper part of the front waist part, or in a case where arms of a person putting on the absorbent article are put in from the leg openings to the waist opening to put on the absorbent article. Thus, the state in which the holes can be visually identified can be easily maintained.

With an absorbent article, wherein preferably the holes are provided in a region, of the waist part on the other side, overlapping the absorbent body in the up-down direction, and the holes are not provided in a region, of the waist part on the other side, not overlapping the absorbent body in the up-down direction.

According to such an absorbent article, the region overlapping the absorbent body in the up-down direction is a region that has little possibility of being held by hands of a person putting on the absorbent article, in a case where the person putting on the absorbent article puts on the absorbent article by holding an upper part of the front waist part, or in a case where arms of a person putting on the absorbent article are put in from the leg openings to the waist opening to put on the absorbent article. Thus, the state in which the holes can be visually identified can be more easily maintained.

With an absorbent article, wherein preferably both end parts of the front-side waist part in the right-left direction and both end parts of the rear-side waist part in the right-left direction are joined to each other, and in a natural state, the front-side waist part and the rear-side waist part are in a protruding shape to the side of the waist part on one side.

According to such an absorbent article, the holes that could not be visually identified due to the creases formed by contraction with a greater contractile force in the natural state are further opened by extending the elastic member when putting on the absorbent article, thus easily drawing attention of the person putting on the absorbent article.

### === Disposable diaper of the present embodiment ===

### <<<Configuration of Disposable diaper 1>>>

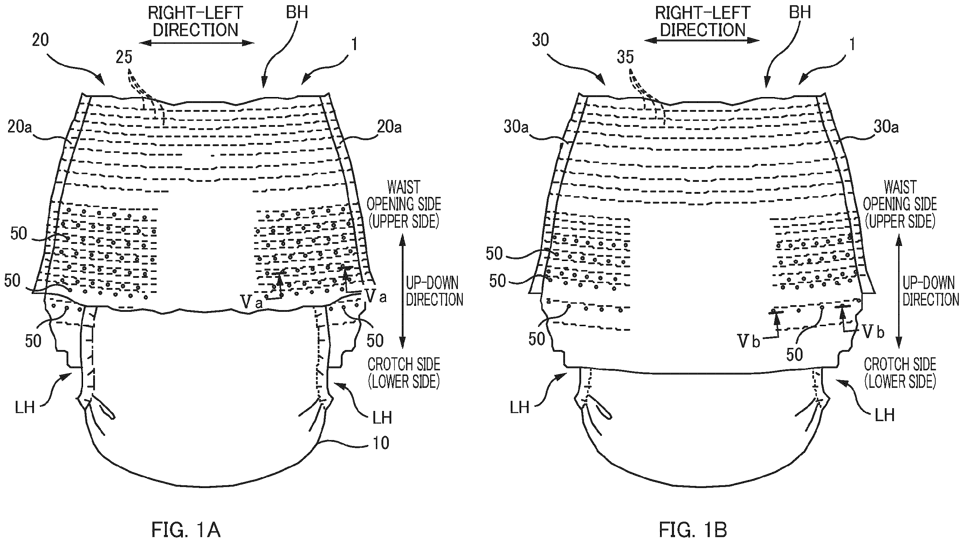

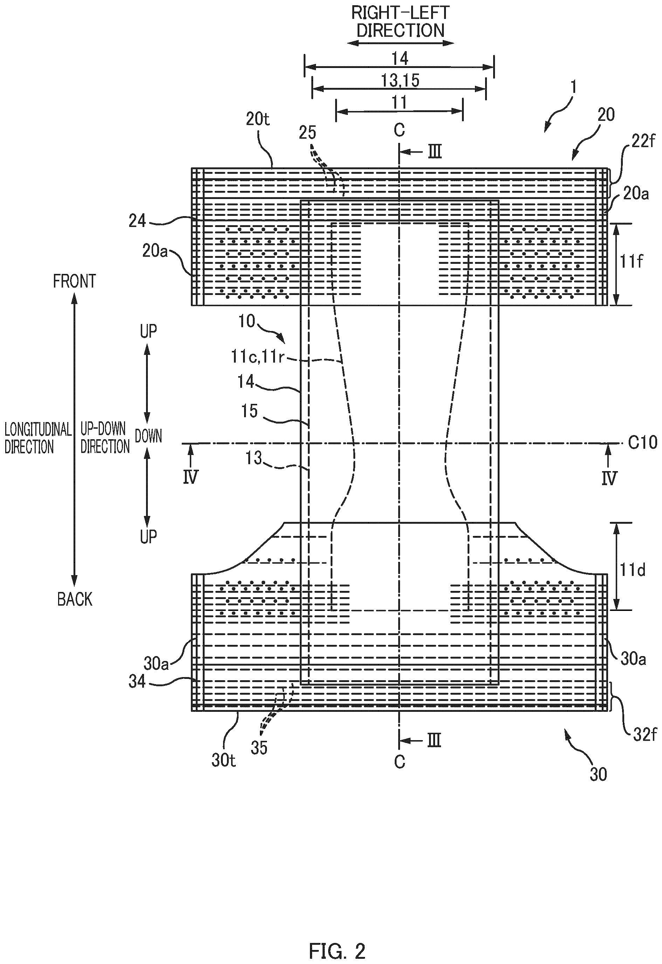

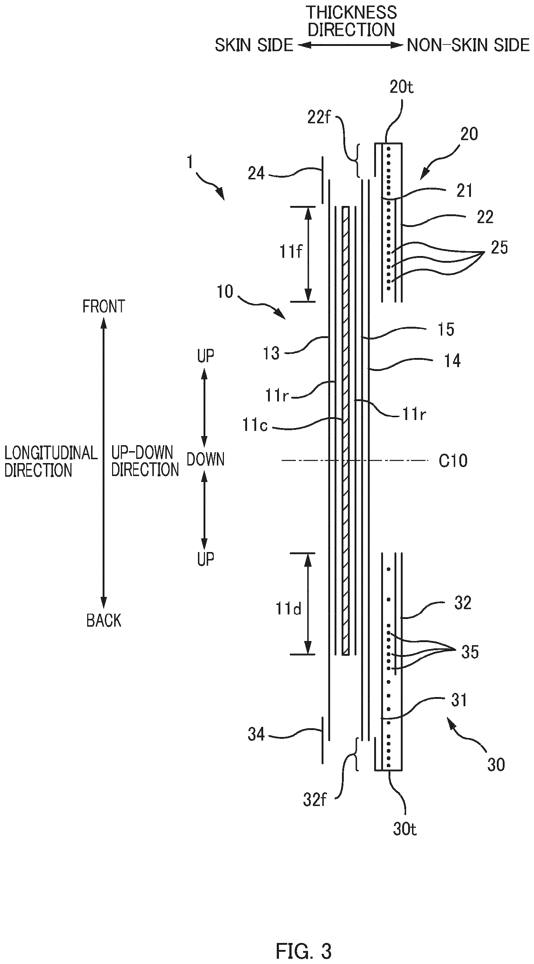

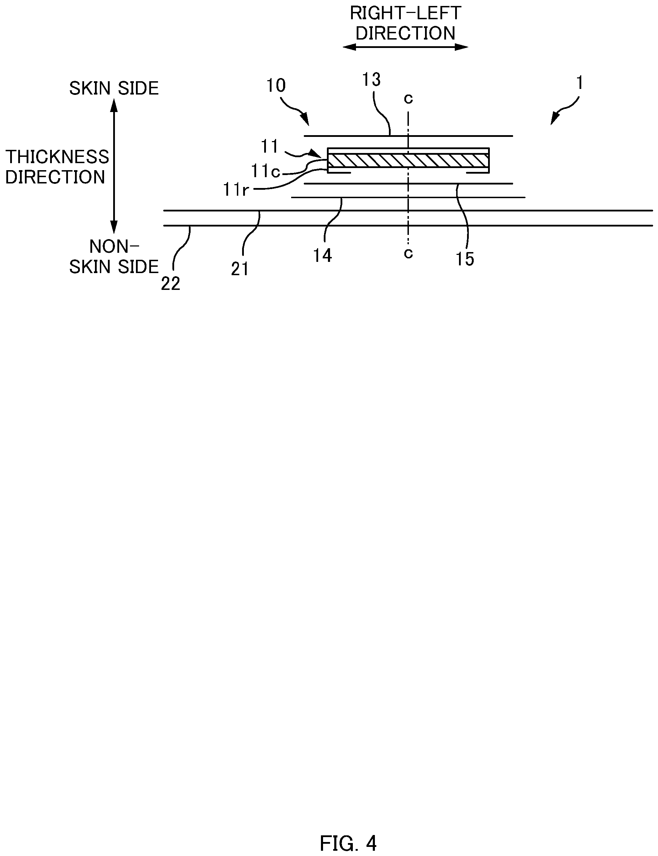

Fig. 1A is a schematic front view of a diaper 1 in a natural state seen from a front side as an example of an absorbent article of the present embodiment. Fig. 1B is a schematic rear view of a diaper 1 in a natural state seen from a rear side. Fig. 2 is a plan view of a diaper 1 in an open extended state seen from a skin side. Fig. 3 is a sectional view along line III-III in Fig. 2. Fig. 4 is a sectional view along line IV-IV in Fig. 2.

In the below description, the diaper 1 in a state in Fig. 1 (a natural state) includes an "up-down direction", a waist opening BH side of the diaper 1 in the up-down direction is referred to as an "upper side" and a crotch side is referred to as a "lower side". The diaper 1 includes a "right-left direction" that intersects with the up-down direction and a "front-rear direction" that intersects with the up-down direction and the right-left direction, and a front side in the front-rear direction is referred to also as "front" and a rear side to as "back". The front-rear direction is also referred to as a "thickness direction", and a side that comes in contact with a wearer is referred to as a "skin side", and an opposite side is referred to as a "non-skin side". A longitudinal direction of the diaper 1 in the state in Fig. 2 (an open state and an extended state) is referred to also as a "longitudinal direction", one side in the longitudinal direction is referred to also as "front", and another side is also referred to as "back". The end on one side in the longitudinal direction and an end on another side are referred to as "upper side", and the approximately central part C10 side in the longitudinal direction is referred to also as a "lower side". Line C-C in Fig. 2 and the like shows a center in the right-left direction.

A "natural state" of the diaper 1 is defined as below. After the diaper 1 that is wrapped as a product is taken out of a package, a front-side waist part 20 and a rear-side waist part 30 are pulled to both outer sides in the right-left direction, and the front-side waist part 20 and the rear-side waist part 30 are extended to a length that matches or is close to a dimension of each independent member. This extended state is maintained for 15 seconds, and then the extended state of the diaper 1 is released and placed on a plane such as a desk. The state of the diaper 1 that has been maintained for 5 minutes in the state placed on the plane is the natural state of the diaper 1. An "open state" is a state where joints of side end parts 20a of the front-side waist part 20 and side end parts 30a of the rear-side waist part 30 are separated and opened to open the entire diaper 1 in a plane. The "extended state" is a state where the diaper 1 (the front-side waist part 20 and the rear-side waist part 30) has been extended until there are no creases, and specifically is a state where the dimension of the members (the front-side waist part 20 and the rear-side waist part 30) configuring the diaper 1 are extended to a length to match or come close to the dimension of the independent members.

The disposable diaper 1 (hereafter referred to as "diaper 1") of this embodiment is namely a three-piece type pull-on diaper that is mainly to be worn by babies and infants. The disposable diaper 1 includes an absorbent main body 10 to be arranged in a crotch of a user, the front-side waist part 20 to cover the front of a wearer, and the rear-side waist part 30 to cover the back of a wearer. Each of the front-side waist part 20 and the rear-side waist part 30 is also referred to as a "waist part". The diaper 1 is put on such as babies and infants by a person such as a parent who puts on the diaper (hereafter, also referred to as a "parent and the like").

As shown in Fig. 2, with the diaper 1 in the open state, the front-side waist part 20 and the rear-side waist part 30 are arranged in parallel with an interval in the longitudinal direction, an absorbent main body 10 is placed over and between these waist parts 20, 30, end parts in a vertical direction of the absorbent main body 10 and each waist part 20, 30 is joined and fixed with such as an adhesive, thus the diaper 1 is in an approximately H shape in planar view. From the open state in the approximately H shape, the absorbent main body 10 is folded in two with an approximately central part C10 in the longitudinal direction as a folding position, and in the folded state, both end parts in the right-left direction of the front-side waist part 20 and the rear-side waist part 30 opposing each other, namely side end parts (also referred to as "end parts") 20a of the front-side waist part 20 and side end parts (also referred to as "end parts") 30a of the rear-side waist part 30 are joined by such as welding. Then, the front-side waist part 20 and the rear-side waist part 30 are joined annularly, to form a pull-on diaper 1 provided with a waist opening BH and a pair of leg openings LH, LH as shown in Fig. 1A and Fig. 1B.

As shown in Fig. 2, Fig. 3, and Fig. 4, the absorbent main body 10 is an approximately rectangular shape in planar view, and the longitudinal direction of the absorbent main body 10 is arranged along the up-down direction. Further, the absorbent main body 10 includes an absorbent body 11, a top sheet 13 that covers the absorbent body 11 from the skin side, an exterior sheet 14 that covers the absorbent body 11 from the non-skin side, and a back sheet 15.

The absorbent body 11 includes a liquid-absorbent absorbent core 11c that absorbs excrements such as urine, and a liquid permeable core-wrapping sheet 11r such as a tissue paper to coat an outer peripheral surface of the absorbent core 11c. The absorbent core 11c is a molded body that is formed by molding a predetermined liquid permeable material into an approximately sand-glass shape in planar view as an example of a predetermined shape. As a liquid absorbent material, liquid absorbent fibers such as pulp fibers and liquid absorbent particles such as superabsorbent polymers (namely SAP) can be given.

The top sheet 13 is a liquid permeable sheet such as a nonwoven fabric of a planar size that protrudes from the absorbent body 11 in the up-down direction and the right-left direction. The back sheet 15 is a sheet with a planar size that protrudes from the absorbent body 11 in the up-down direction and the right-left direction, and is a liquid impermeable leak-proof sheet. A nonwoven fabric exterior sheet 14 is provided to a non-skin side of the back sheet 15. Leg gathers (not shown) that expand and contract in the up-down direction of each of the outer sides in the right-left direction of the absorbent body 11 of the absorbent main body 10 may be provided. Further, to decrease the possibility of excrements leaking from outer sides in the right-left direction of the absorbent main body 10, barrier cuffs (not shown) may be provided as leak-proof wall parts in end parts of the absorbent main body 10 in the right-left direction.

As shown in Fig. 3, the front-side waist part 20 is made by placing and joining the skin side sheet 21 and the non-skin side sheet 22 on the skin side in the thickness direction in order, and the rear-side waist part 30 is made by placing and joining the skin side sheet 31 and the non-skin side sheet 32 on the skin side in the thickness direction in order. The skin side sheet 21, the non-skin side sheet 22, the skin side sheet 31, and the non-skin side sheet 32 are all made of a sheet member approximately rectangular in planar view made of such as spunbond or SMS (spunbond/meltblown/spunbond) nonwoven fabric. In this embodiment, SMS nonwoven fabric is used as the skin side sheets 21, 31, and spunbond nonwoven fabric is used as the non-skin side sheets 22, 32. The spunbond nonwoven fabric may be made of thermoplastic resin single fiber such as polypropylene (PP) or polyethylene (PE) or conjugate fiber such as a PP and PE core-sheath structure.

The skin side sheet 21, the non-skin side sheet 22, the skin side sheet 31, the non-skin side sheet 32 are joined with an adhesive such as a hot-melt adhesive to form the front-side waist part 20 and the rear-side waist part 30.

As shown in Fig. 3, to improve the texture and durability, in an upper side end part of the front-side waist part 20, the non-skin side sheet 22 is folded downward to the skin side, with a front-side upper end 20t that is an upper end of the front-side waist part 20 as the starting point, to form the folded-back section 22f. Similarly, in an upper part of the rear-side waist part 30, the non-skin side sheet 32 is folded downward to the skin side, with a rear-side upper end 30t that is an upper end of the rear-side waist part 30 as the starting point, to form the folded-back section 32f.

The front-side waist part 20 includes a sheet member 24 arranged to cover, from the skin side, a lower end part of the folded-back section 22f to an upper end part of the front of the absorbent main body 10. Similarly, the rear-side waist part 30 includes a sheet member 34 arranged to cover, from the skin side, a lower end part of the folded-back section 32f to an upper end part to the back of the absorbent main body 10. The sheet members 24, 34 are rectangular sheet members made of such as nonwoven fabric. The end parts of the absorbent main body 10 in the up-down direction can be prevented from directly contacting the skin of a wearer with these sheet members 24, 34, and the texture in the waist becomes satisfactory when wearing the diaper. Further, the sheet members 24, 34, can increase the strength of the upper-side end parts of the front-side waist part 20 and the rear-side waist part 30 in the up-down direction.

The front-side waist part 20 and the rear-side waist part 30 are provided with a plurality of approximately circular holes 50 that penetrate from the skin side to the non-skin side with a predetermined interval in between in the up-down direction and the left-right direction. The details of the holes 50 will be described later.

A plurality of elastic members 25, 25 ... such as elastic strings are arranged along the right-left direction between the skin side sheet 21 and the non-skin side sheet 22 of the front-side waist part 20. The elastic members 25 are joined fixedly with such as an adhesive to the skin side sheet 21 and the non-skin side sheet 22 in a state extended in the right-left direction. The plurality of elastic members 25, 25 ... are arranged aligned with an interval in between in the up-down direction.

Similarly, a plurality of elastic members 35, 35 ... such as elastic strings are arranged along the right-left direction between the skin side sheet 31 and the non-skin side sheet 32 of the rear-side waist part 30. The elastic members 35 are joined fixedly with such as an adhesive to the skin side sheet 31 and the non-skin side sheet 32 in a state extended in the right-left direction. The plurality of elastic members 35, 35 ... are arranged aligned with an interval in between in the up-down direction.

The elastic members 25, 35 add elasticity in the right-left direction in respect to the front-side waist part 20 and the rear-side waist part 30, in order to form a plurality of creases to the front-side waist part 20 and the rear-side waist part 30. Many of the creases formed in the front-side waist part 20 and the rear-side waist part 30 shorten each of the sheets 21, 22, 31, 32 in the right-left direction, and the sheets are shaped along the up-down direction.

Regions of the elastic members 25, 35 that overlap with the absorbent body 11 near the central part in the right-left direction are made non-continuous, so that a stretching force does not have an effect. In this way, contraction in the right-left direction that acts on the absorbent body 11 is suppressed, and the absorbent body 11 can be easily maintained in an approximately flat state, thus being able to suppress such as leakage of excrements.

### Holes 50

The holes 50 will be described below. The plurality of holes 50 provided to the front-side waist part 20 and the rear-side waist part 30 each penetrate through the skin side sheets 21, 31 and the non-skin side sheets 22, 32, and improve air permeability from the skin side to the non-skin side, when wearing the diaper 1. Each of the holes 50 in the front-side waist part 20 and the rear-side waist part 30 are approximately circular and approximately the same size. As shown in Fig. 2, in the front-side waist part 20, the plurality of the holes 50 are arranged staggered between the elastic members 25 adjacent in the up-down direction in regions 11f which are to the outer sides of the absorbent main body 10 to the right and left and which overlap with the absorbent body 11 in the up-down direction. In the rear-side waist part 30, the plurality of the holes 50 are arranged staggered between the elastic members 35 adjacent in the up-down direction in regions 11d which are to the outer sides of the absorbent main body 10 in the right and left direction and which overlap with the absorbent body 11 in the up-down direction. Each of the holes 50 does not cut the elastic members 25, 35, and the elastic members 25, 35 are preferably not made non-continuous with the holes 50. The length of the region 11f and the region 11d in the up-down direction of the diaper 1 is approximately the same.

The structure of the holes 50 of the diaper 1 will be specifically described below. "Contractile force" is a force that maintains a contraction state of the front-side waist part 20 or the rear-side waist part 30 when in a natural state. More specifically, in the case where the front-side waist part 20 or the rear-side waist part 30 is each divided into a right half and a left half in an arbitrary position of the diaper 1 in the right-left direction, a force that acts mutually between the right half and the left half is the contractile force. The greater this contractile force, the more contracted the diaper is in the natural state, and the more the creases are formed.

In the case that the length of the front-side waist part 20 and the rear-side waist part 30 in the extended state are the same, in order to decide which waist part has greater contractile force, the one that contracts more in the natural state may be decided as the waist part with the greater contractile force. More specifically, the joints between side end parts 20a of the front-side waist part 20 and side end parts 30a of the rear-side waist part 30 of the pull-on type diaper 1 as shown in Fig. 1A and Fig. 1B are divided, and the front-side waist part 20 and the rear-side waist part 30 are each pulled to both outer sides in the right-left direction, and extended such that the front-side waist part 20 and the rear-side waist part 30 each match or are close to the length of dimensions of each single member. After this extended state is continued for 15 seconds, the extended state is released and the diaper is placed on a flat surface such as a table, and after 5 minutes has passed while being placed on this surface, it is decided which of the front-side waist part 20 and the rear-side waist part 30 has contracted more. With the diaper 1 of this embodiment, because the front-side waist part 20 has contracted more than the rear-side waist part 30, the front-side waist part 20 has a greater contractile force than the rear-side waist part 30, and the rear-side waist part 30 has a smaller contractile force than the front-side waist part 20. The rear-side waist part 30 is also referred to as a "waist part on one side" and the front-side waist part 20 is also referred to as a "waist part on another/other side".

When comparing the size of the contractile force of a tape-type disposable diaper, in the case where only one of the front-side waist part or the rear-side waist part includes the elastic members, of course the waist part with the elastic members can be decided as having a greater contractile force.

Further, "the waist part with the greater contractile force" can also be referred to as a waist part with a smaller value obtained by dividing "a difference between the length in the extended state in the right-left direction and the length in the natural state in the right-left direction" with "the length of the extended state in the right-left direction".

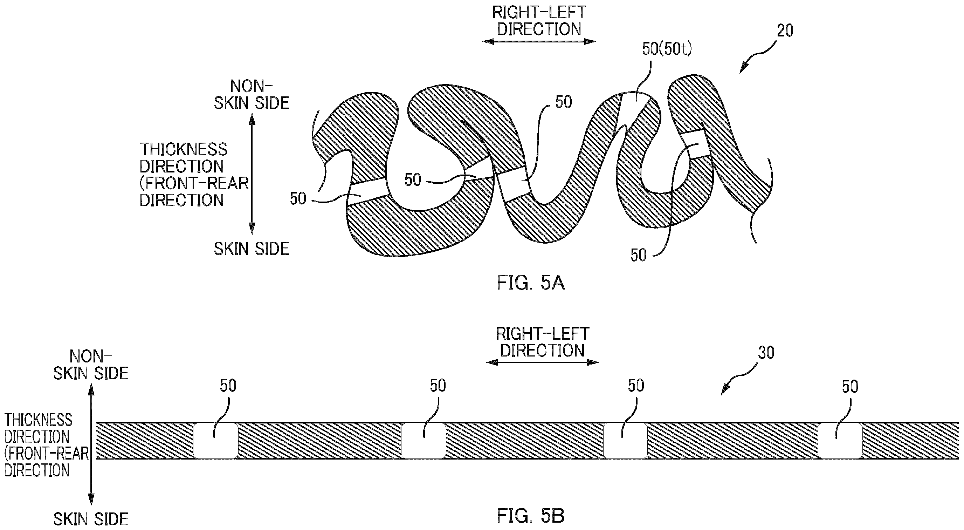

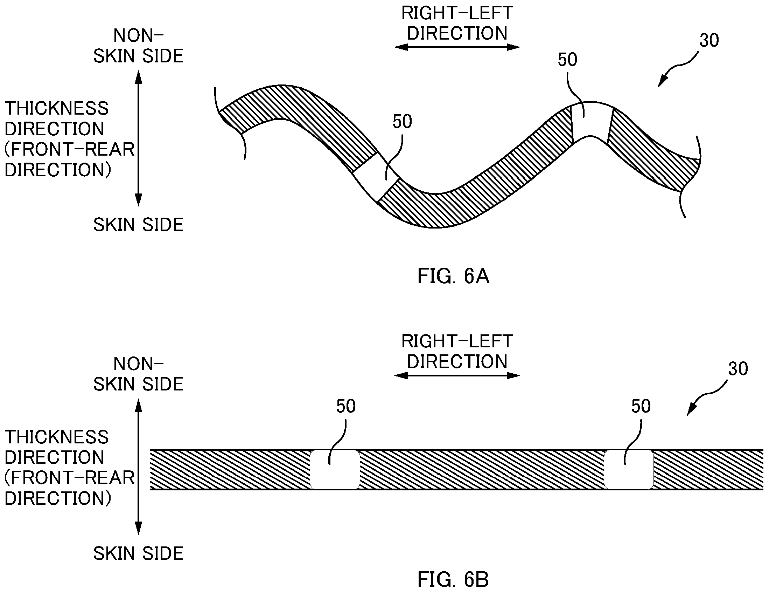

Fig. 5A is a schematic view of a section along Va-Va of the front-side waist part 20 in Fig. 1A, and Fig. 5B is a schematic view of a section along Va-Va extended in the right-left direction from the state in Fig. 5A. Fig. 6A is a schematic view of a section along Vb-Vb of a rear-side waist part 30 in Fig. 1B, and Fig. 6B is a schematic view of a section along Vb-Vb extended in the right-left direction from the state in Fig. 6A. In Fig. 5A, Fig. 5B, Fig. 6A, and Fig. 6B, the skin side sheet 21 and the non-skin side sheet 22, and the skin side sheet 31 and the non-skin side sheet 32 are shown in an integrated manner, and a cross section is shown in a region hatched with lines drawn diagonally to the left lower direction. The section along Va-Va of the front-side waist part 20 in Fig. 1A shown in Fig. 5A and the section along Vb-Vb of the rear-side waist part 30 in Fig. 1B shown in Fig. 6A show a region (unit region) in which the length in the right-left direction is approximately the same.

Usually, with the diaper 1 in the natural state, each of the front-side waist part 20 and the rear-side waist part 30 contracts in the right-left direction due to the contraction of the elastic members 25 and the elastic members 35 in the right-left direction, and the front-side waist part 20 and the rear-side waist part 30 as shown in Fig. 5A and Fig. 6A are formed with a plurality of creases that each rise and fall in the thickness direction.

The diaper 1 has more holes 50 in the waist part (waist part on the other side) that has a greater contractile force in the right-left direction than the waist part (waist part on one side) with a smaller contractile force in the right-left direction. In this embodiment, the front-side waist part (waist part on the other side) 20 that has a greater contractile force in the right-left direction has more holes 50 than the rear-side waist part (waist part on one side) 30 with a smaller contractile force in the right-left direction.

The number of creases per unit region of the front-side waist part 20 that has a greater contractile force is greater than the number of creases per unit region of the rear-side waist part 30 that has a smaller contractile force. With the many formed creases, as shown in Fig. 5A, in the natural state, even in the case where there are a large number of holes 50 per unit region, the holes 50 that are hidden with the creases are provided, and the number of holes 50 that can be visually identified when viewing the front-side waist part 20 from the non-skin side becomes few. Further, the holes 50 are also contracted in the right-left direction, together with contracting of the front-side waist part 20 itself in the right-left direction, and it becomes difficult to visually identify the holes 50.

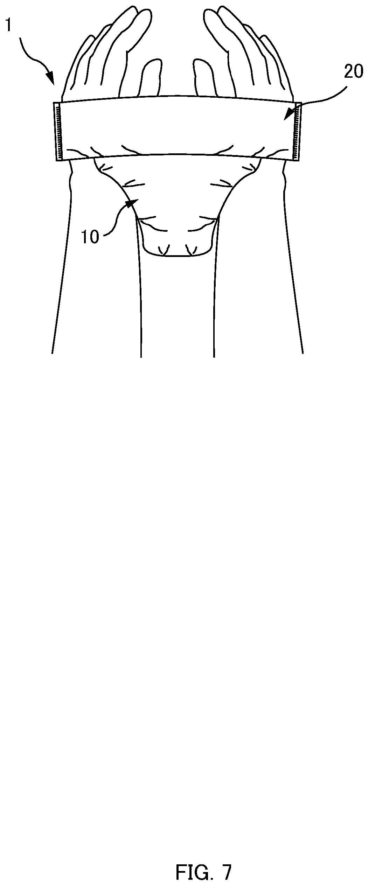

When trying to put on the diaper 1 in the natural state, the parent and the like puts in a baby's or an infant's legs from the waist opening BH, with the waist parts 20, 30 in an extended state to the outside in the right-left direction, and then pulls up the diaper 1. Specifically, there is a method of the parent and the like putting in the legs of a baby or an infant from the waist opening BH by holding the upper part of the waist parts 20, 30 and widening to the outside in the right-left direction, or a method in which the parent and the like puts in her/his arms from the leg openings LH to the waist opening BH and widens the waist parts 20, 30 to the outside in the right-left direction and then puts in the legs of the baby or infant from the waist opening BH. Fig. 7 is a view showing an example of a method of the parent and the like putting on the diaper 1 to a baby or infant.

When the parent and the like extends the waist parts 20, 30 to the outside in the right-left direction, the creases formed in the natural state are stretched in the right-left direction in the front-side waist part 20, to cause the creases to disappear, or the number of the creases to decrease, or the holes 50 to widen in the right-left direction. Fig. 5B shows a state where there are no creases in the section along Va-Va due to the parent and the like having extended the front-side waist part 20 in the right-left direction. In this way, with the diaper 1 in the natural state shown in Fig. 5A, it was difficult to visually identify the holes 50 from the non-skin side, but with the diaper 1 with the front-side waist part 20 extended in the right-left direction as shown in Fig. 5B, the holes 50 can be easily visually identified from the non-skin side. In other words, it is difficult for the parent and the like to notice the presence of the holes 50 provided to the diaper 1, when taking out the diaper 1 from the package, and the parent and the like is not very conscious about air permeability. Then, when the parent and the like puts the diaper 1 on a baby or infant, the holes 50 that could not be seen until then come into view, and can easily surprise the parent and the like. Generally, humans have a nature that the larger the gap with regards to changes of a matter, the more strongly the impression remains. Thus, the more the good air permeability of the diaper 1 in the state taken out from a package is not felt by the parent and the like, the stronger the impression of the holes 50 found when putting on the diaper 1 will be later on, and the easier it will be to make an impression of good air permeability of the diaper 1 to the parent and the like.

In contrast, the number of creases per unit region of the rear-side waist part 20 that has a smaller contractile force is less than the number of creases per unit region of the front-side waist part 20 that has a greater contractile force. Because the number of formed creases is a few, as shown in Fig. 6A, in the natural state, even when the number of the holes 50 per unit region is few, in the case of viewing the rear-side waist part 30 with less creases from the non-skin side, the holes 50 that are hidden with the creases is a few. Contraction of the rear-side waist part 30 in the right-left direction is small compared to contraction of the front-side waist part 20 in the right-left direction, thus contraction of the holes 50 in the right-left direction provided in the rear-side waist part 30 is small, in the natural state, and the holes 50 can be more easily visually identified than the holes 50 provided in the front-side waist part 20.

Similar to the front-side waist part 20, when the parent and the like widens the waist parts 20, 30 to the outside in the right-left direction to put the diaper 1 on the baby or infant, the creases formed in the natural state are stretched in the right-left direction in the rear-side waist part 30, to cause the creases to disappear or the number of the creases to decrease. Fig. 6B shows a state where there are no creases in the section along Vb-Vb due to the rear-side waist part 30 being extended in the right-left direction by the parent and the like. With the diaper 1 in the natural state shown in Fig. 6A, and the diaper 1 with the rear-side waist part 30 extended in the right-left direction as shown in Fig. 6B, the holes 50 can be easily visually identified from the non-skin side. In other words, the parent and the like can easily notice the holes 50 provided to the rear-side waist part 30 of the diaper 1, when taking out the diaper 1, and when the parent and the like is putting the diaper 1 on a baby or infant, the holes 50 that were already recognized in the natural state will be visually identified. Thus, the gap between good air permeability of the diaper 1 that can be recognized in the state when taken out from a package and good air permeability of the diaper 1 that can be seen when putting on the diaper 1 is small, and it is difficult to give the impression of good air permeability.

Although more holes 50 may be formed to improve air permeability, when an extremely large number of the holes 50 are provided in both the front-side waist part 20 and the rear-side waist part 30, there was a possibility that when such as putting on the diaper the holes 50 may tear and the front-side waist part 20 and the rear-side waist part 30 may be damaged.

From the above, when the waist parts 20, 30 are extended in the right-left direction to put on the diaper 1 from the natural state, the holes 50 that could not be visibly identified in the natural state could be visually identified more in the front-side waist part 20 than the rear-side waist part 30. In other words the holes 50 provided in the front-side waist part 20 has a larger number of holes 50 that could be visually identified than the holes 50 provided in the rear-side waist part 30, and the gap in the number of the holes 50 can more effectively easily give the impression of good air permeability to the parent and the like. When considering the possibility of damage in respect to the limited number of holes 50, it is necessary to efficiently arrange the holes 50. By increasing the number of the holes 50 to be provided to the front-side waist part 20, and by decreasing the number of the holes 50 to be provided to the rear-side waist part 30, the issue that the front-side waist part 20 and the rear-side waist part 30 may be damaged can be suppressed.

Because the diaper 1 is provided with more holes 50 in the front-side waist part 20, when putting on the diaper 1 from the front side of a baby or infant, as shown in Fig. 7, the holes 50 in the front-side waist part 20 can be easily visually identified, and good air permeability can be easily recognized.

Further, by providing more holes 50 in the front-side waist part 20 of a pull-on disposable diaper such as the diaper 1, a parent or the like can more easily visually identify the holes 20 more certainly when putting on the diaper 1. For example, in the case of the tape-type disposable diaper, the rear-side waist part with the fastening tapes has elastic members, and the front-side waist part does not usually have elastic members, and the parent and the like generally puts on a diaper to a baby or infant in a facing up state. At this time, even when more holes are provided to the rear-side waist part with greater contractile force than the front-side waist part, the parent and the like may not be able to visually identify the holes in the rear-side waist part that is in a state laid underneath a baby or infant in the facing-up state, when putting on the tape-type diaper. In respect to the above point, with the pull-on diaper 1, by making the contractile force of the front-side waist part 20 greater and providing more holes 50 to the front-side waist part 20, in the case of putting on the diaper from the front side of the baby or infant, and by making the contractile force of the rear-side waist part 30 greater and providing more holes 50 to the rear-side waist part 30, in the case of putting on the diaper from the back side of the baby or infant, the holes 50 will more certainly come into view of the parent and the like, when putting on the diaper 1 to the baby or infant.

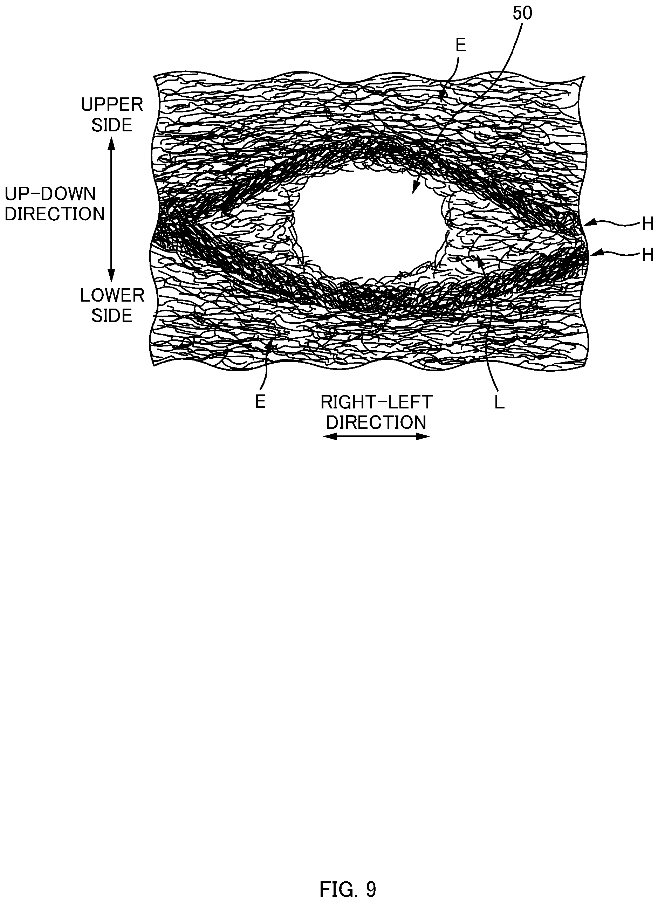

As shown in Fig. 9, a high-density region H is preferably placed to both an upper side and a lower side of the hole 50 in the front-side waist part 20. Fig. 9 is an explanatory view showing an enlarged opening 50. Specifically, the front-side waist part 20 includes, in the up-down direction, the high-density region H between the elastic member 25 adjacent to the hole 50, an outer region E between the high-density region H and the elastic member 25, and a low-density region L between the high-density region H and the hole 50. This high-density region H has a predetermined length, and this predetermined length is longer than the length of the hole 50 in the right-left direction. The density of the fibers of the nonwoven fabric in the high-density region H is higher than the density of the fibers of the nonwoven fabric in the outer region E. For this reason the rigidity of the high-density region is higher than the rigidity of the outer region, and the shape of the hole 50 can be easily maintained, and the hole 50 can be more easily visually identified.

The high-density region H and the outer region E are provided in the forming process of the holes 50. First, the skin-side sheet 21, the elastic members 25, and the non-skin side sheet 22 are overlaid in order and joined fixedly with such as an adhesive. While a first rotary body (not shown) including a plurality of pins and a heat source is rotated, a second rotary body (not shown) including a plurality of holes that engage with the pins and a heat source is rotated in an opposite direction, and as the front-side waist part 20 is heated, the pins of the first rotary body are pressed in toward the front-side waist part 20 and penetrated to engage the holes of the second rotary body, and form the openings 50.

Further, because the front-side waist part 20 is formed with a plurality of creases in the natural state, the front-side waist part 20 is in a state shaped including recesses and protrusions in the front-rear direction (Fig. 5A) . As the hole 50t in Fig. 5A, of the plurality of the holes 50, the hole 50 is preferably formed over a protruding tip to protrude to a non-skin side, namely a tip to the most non-skin side of the creases formed in the front-side waist part 20. The possibility of the high-density region H provided around the edges of the hole 50 directly contacting a skin of a wearer can be decreased, and the texture of the absorbent article can be improved.

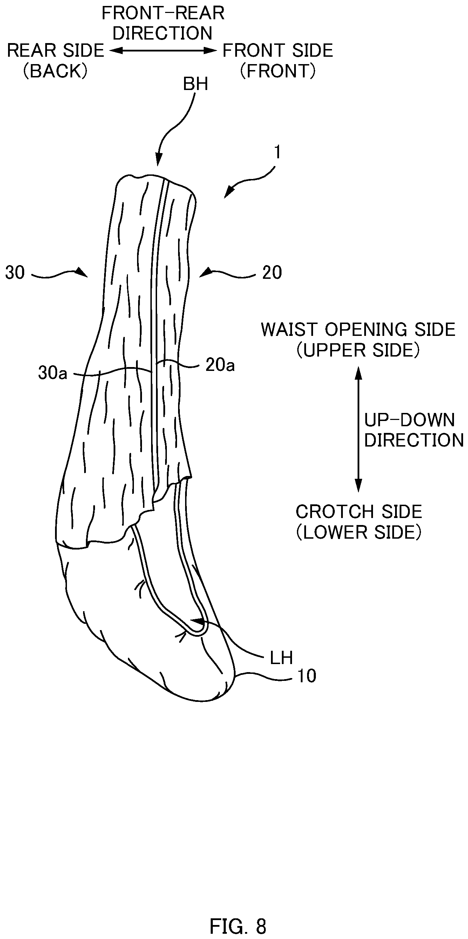

A pull-on diaper 1 may easily be provided with a protruding shape to the front-side waist part 20 side or to the rear-side waist part 30 side because the contractile force of the front-side waist part 20 side and the rear-side waist part 30 are different from each other. Fig. 8 is a schematic side view of the diaper 1 viewed from a one end part side in the right-left direction. Fig. 8 shows the diaper 1 in the natural state, and the diaper 1 in a state that has been taken out of a package is also the same as the above. Because the diaper 1 in the natural state is more contracted in the front-side waist part 20 side that has a greater contractile force, the shape is protruded to the rear-side waist part 30 that has a smaller contractile force. Here, when more holes 50 are included in the front-side waist part 20 with a large contractile force, in the natural state, the front-side waist part 20 side is protruded, and the holes 50 are more difficult to visually identify. But, when the waist parts 20, 30 are extended in the right-left direction to put on the diaper 1, the creases of the front-side waist part 20 are stretched and at the same time the holes 50 are opened in the right-left direction and can be easily visually identified. In this way, the gap in the number and the size of the holes 50 that can be visually identified when in the natural state and when trying to put on the diaper can be easily felt, thus being able to more easily make an impression of good air permeability of the diaper to the parent and the like.

It is preferable that the number of the holes 50 in regions 11f that overlap with the absorbent body 11 in the up-down direction in the front-side waist part 20 is greater than the number of holes 50 in regions that do not overlap with the absorbent body 11 in the up-down direction which is namely regions to the upper side than the regions 11f. It is more preferable that in the front-side waist part 20, the holes 50 are provided in only the regions 11f that overlap with the absorbent body 11 in the up-down direction, and the holes 50 are not provided in regions that do not overlap with the absorbent body 11 in the up-down direction. The regions 11f that overlap with the absorbent body 11 in the up-down direction are regions that have a small possibility of being held with hands of the parent and the like in the case where the parent and the like holds the upper part of the waist parts 20, 30 and puts on the diaper 1, or in the case where arms of the parent and the like are inserted from the leg openings LH to the waist opening BH to put on the diaper 1 as shown in Fig. 7. Thus, the possibility that the holes 50 provided in the regions 11f are pressed or are hidden with hands of the parent and the like can be decreased.

### Other Embodiments

The embodiment of this invention has been described above, and the above embodiment is to facilitate understanding of this invention, and does not limit this invention in any way. It is needless to say that this invention may be changed or modified without departing from the gist thereof and includes its equivalents. A modification as shown below, for example, may be included.

In the above-described embodiment, the holes 50 were formed in both the front-side waist part 20 and the rear-side waist part 30. By forming the holes 50 in both the front-side waist part 20 and the rear-side waist part 30, the air permeability of the diaper 1 can be further improved.

In the above-described embodiment, more holes 50 were formed in the front-side waist part 20 with greater contractile force, but it is not limited to this. The contractile force of the front-side waist part 20 may be made smaller than the rear-side waist part 30, and more holes 50 may be included in the rear-side waist part 30 than the front-side waist part 20. In this way, in the case of putting the diaper 1 on babies and infants lying facing down or babies and infants moving around by crawling, the parent and the like can visually identify the holes 50 formed more in the rear-side waist part 30 when putting the diaper 1 on from the back side of the baby or infant, thus being able to recognize good air permeability.

In the above-described embodiment, the length in the up-down direction of the region 11f in which the front-side waist part 20 and the absorbent body 11 overlap with each other and the length in the up-down direction of the region 11d in which the rear-side waist part 30 and the absorbent body 11 overlap with each other are approximately the same, but it is not limited thereto. For example, the length in the up-down direction of the region 11f in which the front-side waist part 20 with a greater contractile force and the absorbent body overlap with each other may be made longer than the length in the up-down direction of the region 11d in which the rear-side waist part 30 with a smaller contractile force and the absorbent body 11 overlap with each other (11d>11f). The holes 50 included in the region which overlaps in the up-down direction with the absorbent body 11 relatively higher in rigidity than other sections may easily maintain the open shape state even in the natural state. Thus, by making the region 11d>region 11f, when the number of the holes 50 that can be visually identified in the front-side waist part 20 in the natural state are further decreased, and the diaper 1 is put on, more holes that could not be visually identified in the natural state can be visually identified, and the impression of good air permeability can be given to the parent and the like.

In the above-described embodiment, circular holes 50 are provided in the front-side waist part 20 and the rear-side waist part 30, but it is not limited to this. For example, through holes in the shape of a rectangle, oval, or an arbitrary shape such as a star may be provided. Further, the holes 50 of different shapes and different sizes may be provided to the front-side waist part 20 and to the rear-side waist part 30.

The diaper 1 of the above-described embodiment is subject to be worn by babies and infants, but it is not limited to this, and may be worn by adults. Further, in the above-described embodiment, as an example of an absorbent article, namely a three-piece type disposable diaper 1 is exemplified, but it is not limited to this. An absorbent article may be a so-called two-piece type disposable diaper formed with the front-side waist part 20 and the rear-side waist part 30 in an integrated manner, or may be a tape-type disposable diaper.

### Reference Signs List

1 diaper (absorbent article),
10 absorbent main body, 11 absorbent body,
11c absorbent core, 11r core-wrapping sheet,
11f region, 11d region,
13 top sheet, 14 exterior sheet, 15 back sheet,
20 front-side waist part (waist part, waist part on another/other side),
20a side end part (end part), 20t front-side upper end,
21 skin-side sheet, 22 non-skin side sheet,
22f folded-back section,
24 sheet member, 25 elastic member,
30 rear-side waist part (waist part, waist part on one side),
30a side end part (end part), 30t rear-side upper end,
31 skin-side sheet, 32 non-skin side sheet,
32f folded-back section,
34 sheet member, 35 elastic member,
50 hole,
H high-density region, E outer region,
BH waist opening, LH leg opening

## Claims

1. An absorbent article (1) including an up-down direction, a right-left direction, and a front-rear direction, which intersect with each other, comprising:
a front-side waist part (20); and
a rear-side waist part (30),
at least one of the front-side waist part (20) and the rear-side waist part (30) including
an elastic member (25, 35) that expands and contracts in the right-left direction and
holes (50) penetrating in the front-rear direction for improving air permeability when wearing the absorbent article (1), wherein
each of the front-side waist part (20) and the rear-side waist part (30) includes the holes (50),
of the front-side waist part (20) and the rear-side waist part (30), a waist part on another side with a greater contractile force in the right-left direction includes more of the holes than a waist part on one side with a smaller contractile force in the right-left direction, and
the contractile force is a force that maintains a contraction state of the front side wait part (20) or the rear-side waist part (30) when in a natural state, the waist part with the greatest contractile force being measured as described in the description.

2. An absorbent article (1) according to claim 1, wherein
both end parts (20a) of the front-side waist part (20) in the right-left direction and both end parts (30a) of the rear-side waist part (30) in the right-left direction are joined to each other.

3. An absorbent article (1) according to claim 1 or 2, wherein
the waist part on the other side is the front-side waist part (20).

4. An absorbent article (1) according to claim 1 or 2, wherein
the waist part on the other side is the rear-side waist part (30) .

5. An absorbent article (1) according to any one of claims 1 to 4, wherein
the waist part on the other side includes a nonwoven fabric,
in the up-down direction,
a high-density region (H) is included between each hole (50) and the adjacent elastic member (25, 35), and
an outer region (E) is included between the high-density region (H) and the elastic member (25, 35),
the high-density region (H) includes a predetermined length in the right-left direction,
a density of fibers of the nonwoven fabric in the high-density region (H) is higher than a density of fibers of the nonwoven fabric in the outer region (E).

6. An absorbent article (1) according to claim 5, wherein
the waist part on the other side includes a recessed and protruding shape in the front-rear direction, and
the holes (50) are provided to cross over a tip of a protruding part to protrude to a non-skin side.

7. An absorbent article (1) according to any one of claims 1 to 6, wherein
an absorbent body (11) is included, and
a length of a region (11d, 11f) in which the waist part on the other side overlaps with the absorbent body (11), in the up-down direction, is shorter than a length of a region (11d, 11f) in which the waist part on one side overlaps with the absorbent body (11).

8. An absorbent article (1) according to any one of claims 1 to 7, wherein
an absorbent body (11) is included, and
a number of the holes (50) in a region (11d, 11f), of the waist part on the other side, overlapping the absorbent body (11) in the up-down direction is greater than a number of the holes (50) in a region, of the waist part on the other side, not overlapping the absorbent body (11) in the up-down direction.

9. An absorbent article (1) according to claim 8, wherein
the holes (50) are provided in a region (11d, 11f), of the waist part on the other side, overlapping the absorbent body (11) in the up-down direction, and the holes (50) are not provided in a region, of the waist part on the other side, not overlapping the absorbent body (11) in the up-down direction.

10. An absorbent article (1) according to any one of claims 2 to 9, wherein
both end parts (20a) of the front-side waist part (20) in the right-left direction and both end parts (30a) of the rear-side waist part (30) in the right-left direction are joined to each other, and
in a natural state, the front-side waist part (20) and the rear-side waist part (20) are in a protruding shape to the side of the waist part on one side.

## Patentansprüche

1. Absorbierender Artikel (1), der eine Oben-Unten-Richtung, eine Rechts-Links-Richtung und eine Vom-Hinten-Richtung einschließt, die einander schneiden, der Folgendes umfasst:
ein Vorderseitentaillenteil (20) und
ein Hinterseitentaillenteil (30),
wobei mindestens eines von dem Vorderseitentaillenteil (20) und dem Hinterseitentaillenteil (30) Folgendes einschließt:
ein elastisches Element (25, 35), das sich in der Rechts-Links-Richtung dehnt und zusammenzieht, und
Löcher (50), die in der Vom-Hinten-Richtung durchgehen, für die Verbesserung von Luftdurchlässigkeit beim Tragen des absorbierenden Artikels (1), wobei
jedes von dem Vorderseitentaillenteil (20) und dem Hinterseitentaillenteil (30) die Löcher (50) einschließt,
von dem Vorderseitentaillenteil (20) und dem Hinterseitentaillenteil (30) ein Taillenteil auf einer anderen Seite mit einer größeren Kontraktionskraft in der Rechts-Links-Richtung mehr der Löcher einschließt als ein Taillenteil auf einer Seite mit einer kleineren Kontraktionskraft in der Rechts-Links-Richtung und
die Kontraktionskraft eine Kraft ist, die einen Kontraktionszustand des Vorderseitentaillenteils (20) oder des Hinterseitentaillenteils (30) in einem natürlichen Zustand aufrechterhält, wobei das Taillenteil mit der größten Kontraktionskraft gemessen wird, wie in der Beschreibung beschrieben.

2. Absorbierender Artikel (1) nach Anspruch 1, wobei
beide Endteile (20a) des Vorderseitentaillenteils (20) in der Rechts-Links-Richtung und beide Endteile (30a) des Hinterseitentaillenteils (30) in der Rechts-Links-Richtung miteinander verbunden sind.

3. Absorbierender Artikel (1) nach Anspruch 1 oder 2, wobei das Taillenteil auf der anderen Seite das Vorderseitentaillenteil (20) ist.

4. Absorbierender Artikel (1) nach Anspruch 1 oder 2, wobei das Taillenteil auf der anderen Seite das Hinterseitentaillenteil (30) ist.

5. Absorbierender Artikel (1) nach einem der Ansprüche 1 bis 4, wobei
das Taillenteil auf der anderen Seite einen Vliesstoff einschließt,
in der Oben-Unten-Richtung
ein Bereich hoher Dichte (H) zwischen jedem Loch (50) und dem benachbarten elastischen Element (25, 35) eingeschlossen ist und
ein äußerer Bereich (E) zwischen dem Bereich hoher Dichte (H) und dem elastischen Element (25, 35) eingeschlossen ist,
der Bereich hoher Dichte (H) eine vorgegebene Länge in der Rechts-Links-Richtung einschließt,
eine Dichte von Fasern des Vliesstoffs in dem Bereich hoher Dichte (H) höher ist als eine Dichte von Fasern des Vliesstoffs in dem äußeren Bereich (E).

6. Absorbierender Artikel (1) nach Anspruch 5, wobei
das Taillenteil auf der anderen Seite eine vertiefte und herausstehende Form in der Vom-Hinten-Richtung einschließt und
die Löcher (50) bereitgestellt sind, um eine Spitze eines herausstehenden Teils zu überkreuzen, um zu einer Nichthautseite herauszustehen.

7. Absorbierender Artikel (1) nach einem der Ansprüche 1 bis 6, wobei
ein Saugkörper (11) eingeschlossen ist und
eine Länge eines Bereichs (11d, 11f), in dem das Taillenteil auf der anderen Seite mit dem Saugkörper (11) in der Oben-Unten-Richtung überlappt, kürzer ist als eine Länge eines Bereichs (11d, 11), in dem das Taillenteil auf einer Seite mit dem Saugkörper (11) überlappt.

8. Absorbierender Artikel (1) nach einem der Ansprüche 1 bis 7, wobei
ein Saugkörper (11) eingeschlossen ist und
eine Anzahl der Löcher (50) in einem Bereich (11d, 11f) des Taillenteils auf der anderen Seite, der mit dem Saugkörper (11) in der Oben-Unten-Richtung überlappt, größer ist als eine Anzahl der Löcher (50) in einem Bereich des Taillenteils auf der anderen Seite, der nicht mit dem Saugkörper (11) in der Oben-Unten-Richtung überlappt.

9. Absorbierender Artikel (1) nach Anspruch 8, wobei
die Löcher (50) in einem Bereich (11d, 11f) des Taillenteils auf der anderen Seite, der mit dem Saugkörper (11) in der Oben-Unten-Richtung überlappt, bereitgestellt sind und die Löcher (50) nicht in einem Bereich des Taillenteils auf der anderen Seite, der nicht mit dem Saugkörper (11) in der Oben-Unten-Richtung überlappt, bereitgestellt sind.

10. Absorbierender Artikel (1) nach einem der Ansprüche 2 bis 9, wobei
beide Endteile (20a) des Vorderseitentaillenteils (20) in der Rechts-Links-Richtung und beide Endteile (30a) des Hinterseitentaillenteils (30) in der Rechts-Links-Richtung miteinander verbunden sind und
in einem natürlichen Zustand das Vorderseitentaillenteil (20) und das Hinterseitentaillenteil (20) in einer herausstehenden Form zu der Seite des Taillenteils auf einer Seite vorliegen.

## Revendications

1. Article absorbant (1) comprenant une direction allant de haut en bas, une direction allant de droite à gauche, et une direction allant d'avant en arrière, qui se croisent les unes les autres, comportant :
une partie au niveau de la taille côté avant (20) ; et
une partie au niveau de la taille côté arrière (30),
au moins l'une parmi la partie au niveau de la taille côté avant (20) et la partie au niveau de la taille côté arrière (30) comprenant
un élément élastique (25, 35) qui s'étend et se contracte dans la direction allant de droite à gauche et
des trous (50) qui pénètrent dans la direction allant d'avant en arrière à des fins d'amélioration de la perméabilité à l'air lors du port de l'article absorbant (1), dans lequel
chacune parmi la partie au niveau de la taille côté avant (20) et la partie au niveau de la taille côté arrière (30) comprend les trous (50),
parmi la partie au niveau de la taille côté avant (20) et la partie au niveau de la taille côté arrière (30), une partie au niveau de la taille sur un autre côté avec une force de contraction supérieure dans la direction allant de droite à gauche comprend un plus grand nombre de trous par rapport à une partie au niveau de la taille sur un côté avec une force de contraction inférieure dans la direction allant de droite à gauche, et
la force de contraction est une force qui maintient un état de contraction de la partie au niveau de la taille côté avant (20) ou de la partie au niveau de la taille côté arrière (30) quand dans un état naturel, la partie au niveau de la taille ayant la force de contraction la plus grande étant mesurée tel qu'il est décrit dans la description.

2. Article absorbant (1) selon la revendication 1, dans lequel
les deux parties d'extrémité (20a) de la partie au niveau de la taille côté avant (20) dans la direction allant de droite à gauche et les deux parties d'extrémité (30a) de la partie au niveau de la taille côté arrière (30) dans la direction allant de droite à gauche sont assemblées les unes par rapport aux autres.

3. Article absorbant (1) selon la revendication 1 ou la revendication 2, dans lequel
la partie au niveau de la taille sur l'autre côté est la partie au niveau de la taille côté avant (20).

4. Article absorbant (1) selon la revendication 1 ou la revendication 2, dans lequel
la partie au niveau de la taille sur l'autre côté est la partie au niveau de la taille côté arrière (30).

5. Article absorbant (1) selon l'une quelconque des revendications 1 à 4, dans lequel
la partie au niveau de la taille sur l'autre côté comprend un tissu non tissé,
dans la direction allant de haut en bas,
une région haute densité (H) est incluse entre chaque trou (50) et l'élément élastique adjacent (25, 35), et
une région extérieure (E) est incluse entre la région haute densité (H) et l'élément élastique (25, 35),
la région haute densité (H) comprend une longueur prédéterminée dans la direction allant de droite à gauche,
une densité de fibres du tissu non tissé dans la région haute densité (H) est supérieure par rapport à une densité de fibres du tissu non tissé dans la région extérieure (E).

6. Article absorbant (1) selon la revendication 5, dans lequel
la partie au niveau de la taille sur l'autre côté comprend une forme en retrait et faisant saillie dans la direction allant d'avant en arrière, et
les trous (50) sont mis en œuvre pour croiser un bout d'une partie faisant saillie pour faire saillie sur un côté non orienté vers la peau.

7. Article absorbant (1) selon l'une quelconque des revendications 1 à 6, dans lequel
un corps absorbant (11) est inclus, et
une longueur d'une région (11d, 11f) dans laquelle la partie au niveau de la taille sur l'autre côté chevauche le corps absorbant (11), dans la direction allant de haut en bas, est plus courte par rapport à une longueur d'une région (11d, 11f) dans laquelle la partie au niveau de la taille sur un côté chevauche le corps absorbant (11).

8. Article absorbant (1) selon l'une quelconque des revendications 1 à 7, dans lequel
un corps absorbant (11) est inclus, et
un certain nombre de trous (50) dans une région (11d, llf), de la partie au niveau de la taille sur l'autre côté, chevauchant le corps absorbant (11) dans la direction allant de haut en bas est supérieur par rapport à un certain nombre de trous (50) dans une région, de la partie au niveau de la taille sur l'autre côté, ne chevauchant pas le corps absorbant (11) dans la direction allant de haut en bas.

9. Article absorbant (1) selon la revendication 8, dans lequel
les trous (50) sont mis en œuvre dans une région (11d, llf), de la partie au niveau de la taille sur l'autre côté, chevauchant le corps absorbant (11) dans la direction allant de haut en bas, et les trous (50) ne sont pas mis en œuvre dans une région, de la partie au niveau de la taille sur l'autre côté, ne chevauchant pas le corps absorbant (11) dans la direction allant de haut en bas.

10. Article absorbant (1) selon l'une quelconque des revendications 2 à 9, dans lequel
les deux parties d'extrémité (20a) de la partie au niveau de la taille côté avant (20) dans la direction allant de droite à gauche et les deux parties d'extrémité (30a) de la partie au niveau de la taille côté arrière (30) dans la direction allant de droite à gauche sont assemblées les unes par rapport aux autres, et
dans un état naturel, la partie au niveau de la taille côté avant (20) et la partie au niveau de la taille côté arrière (20) sont en une forme faisant saillie sur le côté de la partie au niveau de la taille sur un côté.
